# EUROPEAN PATENT SPECIFICATION

(11) **EP 0 671 003 B1**
(45) Date of publication and mention of the grant of the patent: **29.01.1997**
(21) Application number: 93922561.1
(22) Date of filing: 12.10.1993
(51) Int. Cl.: G01N 33/28, G01N 21/35

(54) **METHOD FOR PREDICTION OF CETANE NUMBERS OF GASOILS**
VERFAHREN ZUR VORAUSSAGE DER CETANZAHLEN VON GASÖLEN
PROCEDE DE PREDICTION D'INDICES DE CETANE DE GAZOLES

(30) Priority: 15.10.1992 EP 92309405
(43) Date of publication of application: 13.09.1995
(73) Proprietor: SHELL INTERNATIONALE RESEARCH MAATSCHAPPIJ B.V., 2596 HR Den Haag (NL)
(72) Inventor: BOYD, Andrew, Chester, Cheshire CH1 3SH (GB); GILCHRIST, Catherine Anne, Chester, Cheshire CH1 3SH (GB); TOLCHARD, John Michael, Chester, Cheshire CH1 3SH (GB)
(86) International application number: EP9302835
(87) International publication number: WO9409362

(56) References cited:
- EP-A- 0 305 090
- WO-A-91/15762
- DATABASE WPI Week 9133, 10 July 1991 Derwent Publications Ltd., London, GB; AN 91-243856 ANON. 'DIRECT DETERMN. OF LIQUID HYDROCARBON FUEL PROPERTIES - BY USING NEURAL NETWORK TO ANALYSE BY IR SPECTROSCOPIC DATA OBTD. FROM THE FUEL FOR DETECTION OF ANY CONTAMINATION IN BULK FUEL'

## Description

The present invention relates to a method for prediction of cetane number of a gasoil by correlation of its (near) infra-red ((N).I.R.) spectrum to the cetane number. As known to those skilled in the art, organic compounds have in the infra-red region (about 1 to about 300 µm) a unique spectral fingerprint.

Recent market trends for bulk fuels put more onus on the quality and performance characteristics of the product. Whole marketing campaigns are being fought to convince the customer that one fuel noticeably performs better than any other, and this philosophy is expected to continue. Such an approach needs to be backed up by full quality assurance, from refining through blending and finally to delivery to the customer. This requirement can be best met by using quality monitoring instruments to measure performance characteristics of the fuel.

At the moment, after leaving the refinery there are generally no continuous, quantitative checks made on the fuel to see if it still is within specification: it is only manually checked for clarity, smell and has its density noted. This, taking into account the highly complex nature of the modern advanced fuels is not enough. A quality monitoring instrument package which actually measures performance specification of fuels would be advantageous, so degradation to the fuel, cross contamination with other fuels, and changes in the fuels composition could be detected and pinpointed in the distribution chain. This will not only ensure the customer receives the fuel exactly as intended, but problems along the distribution chain may be found and addressed.

An important performance measure of a fuel such as gasoil is cetane number which is a quantity reflecting the ignition properties of diesel oil. Engines do exist for cetane number measurement.

Reference fuels are used in the same way as for the octane number determination for motor gasoline. These reference fuels are n-cetane and α-methyl naphthalene. The ignition quality is expressed as the cetane number, which is the percentage by volume of cetane in a blend with α-methyl naphthalene whose ignition performance matches that of the fuel in the test engine. In practice, cetane engines are seldom used, and cetane is normally calculated as "cetane index" from other measurements e.g. density and distillation. The cetane index, however, may not be suitable for diesel fuels of the future, and an alternative measurement of cetane number is required.

Further, it has already been proposed to determine the cetane number of gasoil as a function of near-infra-red (N.I.R) spectra. (Vide e.g. EP-A-0,304,232.)

However, until now it was not possible to predict the cetane number of a gasoil from its I.R. spectrum (including near-infra-red and mid-infra-red) (advantageously, 0.78-30 µm wavelength is applied).

It is therefore an object of the invention to provide a method for prediction of cetane number of unknown gasoils, wherein the cetane number of blended product and of process streams with a wide range of cetane numbers is predicted.

The invention therefore provides a method for prediction of cetane numbers of gasoils, comprising the steps of:
a) measuring the I.R. spectra of a plurality of sets of gasoils;
b) selecting in the spectral region a range of wave numbers; and converting a number of the wavelengths in question to absorption data and using said absorption data as an input to a neural network; characterized by:
c) analyzing the spectral data using a neural network, wherein the number of layers of the neural network is 2 to 5, and, in case of three or more layers, the number of nodes of the input layer is from 3 to 10, the number of nodes of the hidden layer(s) is from 1 to 10, and the number of nodes of the output layer is from 1 to 3;
d) determining cetane number of the gasoil by conventional measurement;
e) selecting a training data set comprising measured I.R. data and conventionally measured cetane number data and correlating the obtained absorbance values with cetane number, generating a set of predictive data; and subsequently
f) applying these data to infra-red spectra, taken under the same conditions, for gasoils of unknown cetane number, thus providing the cetane number of the unknown gasoil.

As already indicated in the foregoing, the invention is based upon the principle of correlation of cetane number of a gasoil to its infra-red spectrum. This principle is known as such (e.g. from EP-A-0,304,232 and EP-A-0,285,251) and will not be described in detail. It is remarked that RD 327135 discloses the use of a neural network for determining octane number of a gasoline. However, the specific neural networks for determining cetane number according to the present invention have not been suggested.

According to the invention the infra-red spectra of a large set of gasoils (advantageously at least 100), from a wide variety of sources are measured. Components from different secondary conversion processes may be present.

The variety is important since this determines the generality and applicability of any subsequent statistical predictive tool.

The spectral region used is 9000-4500 wave numbers for finished and fully blended gasoils. A measurement of the cetane ignition improver concentration is also required (1600-1700 wavenumbers, advantageously 1630 wave number), because this influences the cetane number.

For work on conversion process streams, for real-time process control application, the spectral region is also 9000-4500 wave numbers.

The spectra are then analyzed using a neural network, together with determinations of cetane number by conventional test engine measurement, using multivariate statistical techniques known as such, e.g. Partial Least Squares, Multiple Linear Regression, Reduced Rank Regression, Principle Components Analysis and the like.

Subsequently predictive data are generated that can subsequently be applied to the infra-red spectra, taken under the same conditions, for gasoils of unknown cetane number.

Standard errors of prediction have been achieved less than 0-4 cetane numbers.

The general theory and general operation of neural networks as such is known to those skilled in the art and will therefore not be described in detail.

Generally, a neural network can be defined as a system, wherein during a learning period a correlation between input- and output variables is searched for. After sufficient examples have been offered in this learning period the neural network is able to produce the relevant output for an arbitrary input. Neural networks have found applications e.g. for pattern recognition problems.

As those skilled in the art will appreciate, neural networks are built up of layers of processing elements (similar to the brain's neurons) each of which is weighted and connected to elements in other layers (similar to the brain's synapses). A network learns patterns by adjusting weights between the elements whilst it is being trained with accurate qualified data.

According to an advantageous learning algorithm, training errors, the difference between the actual and predicted result are propagated backwards through the network to the hidden layers which receive no feedback from training patterns. The weights of the interconnections are adjusted in small steps in the direction of the error, to minimize the errors, and the training data is run through again. This happens many times till the error reaches an acceptable level, which is usually the repeatability of the initial measurement.

As already indicated in the foregoing, multivariate statistical techniques for data analysis are known as such, but for reasons of clarity here a more detailed description of the analysis of infra-red spectral data using Principal Component Analysis is provided.

In the matter of the above prediction, a training set is defined as those gasoils whose spectra and 'engine-measured' cetane values are used in the analysis and generation of predictive data, which can subsequently be used to determine the cetane number of an unknown gasoil. The selection of this training set is clearly important because it critically influences the generality and predictive performance of the data. Data analysis on the set of spectra corresponding to the gasoils of the training set is done in the following manner:
1. The mean spectrum of the set is generated and the differences between each individual spectrum and the mean are calculated.
2. The mean spectrum will be in the order of 5000 data points and so the problem of analysis of a set of 100 gasoils is very difficult. A technique is required to allow data reduction to a manageable number of problem variables. A set of artificial spectra are calculated by simultaneous analysis of the difference spectra such that the variance in the data can be explained by these artificial spectra. The difference spectra are defined here as the arithmetic differences in absorbance between the mean spectrum of the gasoil set and each individual gasoil spectrum. The artificial spectra are known as the principal components and, when added in correct proportion to the mean spectrum, re-generate the individual gasoil spectra. For example a typical number of principal components would be 10 and so the original gasoil spectra can be re-generated by addition of appropriate contributions of each principal component to the mean. The contribution due to each principal component is known as the principal component score and the scores will be different for each gasoil.
3. The information in the gasoil spectra has thus been reduced from 5000 data points for each to 10 principal component scores. These scores can be correlated with the engine-measured cetane numbers using a technique such as multiple linear regression.
4. The subsequent analysis of an unknown gasoil involves the measurement of its spectrum and then spectral description in terms of the principal components used before to describe the training set of gasoil spectra. This results in a set of the principal component scores for the unknown gasoil. The coefficients of the earlier multiple linear regressional correlation can then be applied to the principal component scores of the unknown gasoil's spectrum, providing the cetane number of the unknown gasoil.
5. The analysis in the case of the neural network technology is different from the above, because the data reduction is performed by physical reduction in the number of measured wavelengths. The data reduction is in the following manner: Principal Component Analysis is used on the training set of gasoils, to generate a 'property spectrum' which represents the relative importance of each spectral data point to the correlation with cetane number. The spectral measurement is then simplified to discrete wavelengths, typically numbering between 5 and 10.

One of the wavelengths is advantageously used as a transmission reference to correct for any instrumental drifts.

The remaining wavelengths, corrected by the reference, are converted to absorption data. This may be done logarithmically, and the data can be mathematically scaled within predetermined bounds for each wavelength. That is, extreme values expected for either fuels, or more likely, process streams are used to provide the range of acceptable absorbances at each wavelength against which the scaling can be done for the fuel to be tested. The absorbance values are used as the input to the neural network. The neural network performs pattern recognition tasks and the application here is clearly to recognise the pattern in the spectral data with relationship to the cetane number. Before the neural network is used to predict cetane number of unknown gasoils, it must be 'trained'. The manner of training is to select the 'training set' to a network, whose size and architecture have been designed to match the recognition problem. During the training phase the neural network is repeatedly presented with the infra-red data and the engine-measured cetane numbers for each of the gasoils, and the relationship is 'learned'. The manner of the 'learning' is that upon each presentation of the infra-red data there is a prediction, by the neural network, of cetane number which is compared to the engine value and the difference is used as feedback to correct the neural network to its next prediction. Once the neural network has "learned" the relationship, the data set should be split into a further training set and a validation set that will not be used in the "learning" phase. This process continues until the network has 'learned' sufficiently, and the predictive errors are comparable to the uncertainties in the original cetane number determination by engine. Once this phase is complete, the neural network can be used to predict cetane number of an unknown gasoil, after measurement of infra-red spectral data at the wavelengths used during the training.

In particular, the said predictive data is generated by training the neural network on the entire data set by repeated presentation of inputs and known outputs i.e. the infra-red data for the gasoil and its relevant cetane number data, to learn the relationship between the two, and monitoring the performance of its predictions against the actual relevant cetane number data as measured by standard methods for the training data, thus correlating the absorbance values with cetane number; generating a set of values of the interconnection weights and biases of the network as adjusted after the said learning period; and applying these adjusted values, utilizing the network algorithm, to infra-red spectra, taken under the same conditions, for gasoils of unknown cetane number.

Advantageously, the network used has a three-layer architecture which, for example, comprises in a first layer four input nodes, 2 hidden nodes in a second layer between the input and output, and in a third layer one output node. This is called a (4, 2, 1) network. The spectral data are presented as inputs to the input nodes, wherein the product quality information is the output.

As known to those skilled in the art the nodes possess certain weights of interconnections, and may be biased.

The weights and biases of the network can be stored and used to analyze input data comprising the measured infra-red absorbances and correlate the pattern to the cetane number of a gasoil. Thus, for a prediction which utilizes the network algorithm to describe cetane number from I.R.-data, important parameters, having been trained and successfully tested against the validation set, are the weights of interconnection between the nodes and the biases at the hidden and output nodes.

These can be interrogated and then implemented in the network algorithm for the cetane number analysis of future fuel samples.

For multiple outputs, a neural network algorithm is implemented for each output. The implementation is by software code on a microprocessor chip, and is therefore flexible to any changes in network parameters which can be easily re-programmed.

It will be appreciated by those skilled in the art that the network architectures applied may vary in the precise number of nodes that are present in each layer, or even in the number of actual layers. Advantageously, 2 to 5 layers are applied.

According to the invention advantageously the number of nodes of the input layer ranges from 3-10, the number of nodes of the hidden layer(s) ranges from 1-10, and the number of nodes of the output layer ranges from 1-3. More in particular, (3, 5, 1), (6, 6, 3) and (6, 6, 6, 3) networks could be applied.

The method of the invention will now be described by reference to the following examples:

### Example A

A training set of 20 blended gasoils, collected from different refineries were measured in the infra-red spectral region 9000-4500 wave numbers. The samples were all rated for cetane numbers using the ASTM D613 method which uses a 'cetane engine'. The training set was analyzed in the manner described before. The analysis using the principal components to describe the data by 'artificial spectra' is done, and provides the first three principal component spectra in the range 6400-4800 wave numbers. The training set used in this example required 5 principal components, and the results showed a correlation between the engine-measured cetane numbers and the infra-red prediction of cetane numbers of 0.94, and standard errors of prediction of less than 0.4 cetane numbers. The use of neural networks for the prediction of cetane number instead of the above analysis begins, for the first training set, with principal component analysis to generate a property spectrum from which the important, discrete wavelengths can be identified. The neural network approach subsequently used would start with the measurement of the training set infra-red data at those wavelengths identified in this analysis.

### Example B

A training set of 11 gasoil blending components e.g. light cat cracked cycle oil, straight run distillate, with cetane numbers in a broad range between 20 and 60, is used to demonstrate the breadth of applicability of infra-red cetane determination. The infra-red spectra are measured in the range 9000-4500 wave numbers. A predictive data which uses 3 principal components is generated in the manner of the stated procedure. A correlation coefficient of 0.99 between the predicted values and the measured values of cetane number is provided. Cross-validation provides a good indicator of predictive performance on unknown fuels. The set of data in this example gives results with standard errors of prediction of better than 0.9. This performance is lower than the previous example, and reflects the smaller training set and broader cetane number range of the fuels. A larger training set would improve this figure to comparable levels as example A.

Various modifications of the invention will become apparent to those skilled in the art from the foregoing description and accompanying drawings. Such modifications are intended to fall within the scope of the appended claims.

## Claims

1. A method for prediction of cetane numbers of gasoils, comprising the steps of:
a) measuring the I.R. spectra of a plurality of sets of gasoils;
b) selecting in the spectral region a range of wave numbers; and converting a number of the wavelengths in question to absorption data and using said absorption data as an input to a neural network; characterized by:
c) analyzing the spectral data using a neural network, wherein the number of layers of the neural network is 2 to 5, and, in case of three or more layers, the number of nodes of the input layer is from 3 to 10, the number of nodes of the hidden layer(s) is from 1 to 10, and the number of nodes of the output layer is from 1 to 3;
d) determining cetane number of the gasoil by conventional measurement;
e) selecting a training data set comprising measured I.R. data and conventionally measured cetane number data and correlating the obtained absorbance values with cetane number, generating a set of predictive data; and subsequently
f) applying these data to infra-red spectra, taken under the same conditions, for gasoils of unknown cetane number, thus providing the cetane number of the unknown gasoil.

2. The method as claimed in claim 1, wherein the spectral range is 0.78-30 µm wavelength.

3. The method as claimed in claim 1 or 2, wherein the set of gasoils is at least 100.

4. The method as claimed in any one of claims 1-3, wherein the sets of gasoils contain components from different secondary conversion processes.

5. The method as claimed in any one of claims 1-4, wherein the spectral region used is 9000-4500 wave numbers for finished and fully blended gasoils and the cetane ignition improver concentration is measured in the spectral region of 1600-1700 wave numbers.

6. The method as claimed in any one of claims 1-4, wherein the spectral region is 9000-4500 wave numbers for conversion process streams with a range of cetane numbers.

7. The method as claimed in any one of claims 1-6, wherein a neural network is applied, comprising the steps of:
training the neural network on the entire data set by repeated presentation of inputs and known outputs i.e. the near infra-red data for the gasoil and its cetane number, to learn the relationship between the two, and monitoring the performance of its predictions against the actual cetane number data as measured by standard methods for the training data, thus correlating the absorbance values with said cetane number;
generating a set of values of the interconnection weights and biases of the network as adjusted after the said learning period; and
applying these adjusted values, utilizing the network algorithm to near infra-red spectra, taken under the same conditions, for gasoils of unknown cetane number.

8. The method as claimed in any one of claims 1-7, wherein the network comprises 4 input nodes, 2 hidden nodes and one output node ((4, 2, 1) network).

9. The method as claimed in any one of claims 1-7, wherein the network is a (3, 5, 1) network.

10. The method as claimed in any one of claims 1-7, wherein the network is a (6, 6, 3) network.

11. The method as claimed in any of claims 1-7, wherein the network is a (6, 6, 6, 3) network.

## Patentansprüche

1. Verfahren zur Vorhersage der Cetanzahlen von Gasölen, mit den Schritten:
a) Messen des Infrarotspektrums einer Mehrzahl von Sätzen von Gasölen;
b) Auswählen eines Bereiches von Wellenzahlen im Spektralbereich; und Umwandeln einer Anzahl der in Rede stehenden Wellenlängen in Absorptionsdaten und Verwenden der Absorptionsdaten als Eingabe für ein neuronales Netzwerk; gekennzeichnet durch:
c) Analysieren der Spektraldaten unter Verwendung eines neuronalen Netzwerkes, wobei die Anzahl der Schichten des neuronalen Netzwerkes 2 bis 5 beträgt, und, im Falle von 3 oder mehr Schichten, die Anzahl von Knoten der Eingangsschicht 3 bis 10 beträgt, die Anzahl der Knoten der verborgenen Schicht(en) 1 bis 10 beträgt, und die Anzahl der Knoten der Ausgangsschicht 1 bis 3 beträgt;
d) Bestimmen der Cetanzahl des Gasöls durch herkömmliche Messung;
e) Auswählen eines Trainingsdatensatzes mit den gemessenen Infrarotdaten und herkömmlich gemessenen Cetanzahldaten und Korrelieren der erhaltenen dekadischen Extinktionswerte mit der Cetanzahl, Erzeugen eines Satzes von Voraussagedaten; und anschließendes
f) Anwenden dieser Daten auf Infrarotspektren, die unter denselben Bedingungen für Gasöle unbekannter Cetanzahl aufgenommen wurden, um dadurch die Cetanzahl eines unbekannten Gasöls zu erhalten.

2. Verfahren nach Anspruch 1, bei welchem der Spektralbereich die Wellenlänge 0,78 bis 30 µm ist.

3. Verfahren nach Anspruch 1 oder 2, bei welchem der Satz von Gasölen zumindest 100 ist.

4. Verfahren nach einem der Ansprüche 1-3, bei welchem die Sätze von Gasölen Komponenten aus unterschiedlichen Sekundärumwandlungsprozessen enthalten.

5. Verfahren nach einem der Ansprüche 1-4, bei welchem der verwendete Spektralbereich die Wellenzahlen 9000-4500 für fertige und vollständig gemischte Gasöle umfaßt und die Cetanzündungsverbesserungskonzentration in dem Spektralbereich der Wellenzahlen 1600-1700 gemessen wird.

6. Verfahren nach einem der Ansprüche 1-4, bei welchem der Spektralbereich die Wellenzahlen 9000-4500 für Umwandlungsprozeßströme mit einem Bereich von Cetanzahlen umfaßt.

7. Verfahren nach einem der Ansprüche 1-6, bei welchem ein neuronales Netzwerk angewandt wird, mit den Schritten:
Trainieren des neuronalen Netzwerkes auf den gesamten Datensatz durch wiederholtes Präsentieren von Eingaben und bekannten Ausgaben, d.h. Nahe-Infrarotdaten für das Gasöl und seine Cetanzahl, um die Beziehung zwischen diesen beiden zu lehren, und Überwachen der Leistung seiner Vorhersage gegenüber den tatsächlichen Cetanzahldaten, wie sie durch Standardverfahren für die Trainingsdaten gemessen werden, und dadurch Korrelieren der dekadischen Extinktionswerte mit dieser Cetanzahl;
Erzeugen eines Satzes von Werten der gegenseitigen Verbindungsgewichtungen und Abgleiche des Netzwerkes, wie es nach der Lernphase eingestellt ist; und
Anwenden dieser eingestellten Werte unter Verwendung des Netzwerkalgorithmus auf Nahe-Infrarotspektren, die unter denselben Bedingungen aufgenommen wurden, für Gasöle unbekannter Cetanzahl.

8. Verfahren nach einem der Ansprüche 1-7, bei welchem das Netzwerk 4 Eingangs knoten, 2 verborgene Knoten und einen Ausgangsknoten aufweist ((4, 2, 1)-Netzwerk).

9. Verfahren nach einem der Ansprüche 1-7, bei welchem das Netzwerk ein (3, 5, 1)-Netzwerk ist.

10. Verfahren nach einem der Ansprüche 1-7, bei welchem das Netzwerk ein (6, 6, 3)-Netzwerk ist.

11. Verfahren nach einem der Ansprüche 1-7, bei welchem das Netzwerk ein (6, 6, 6, 3)-Netzwerk ist.

## Revendications

1. Procédé pour la prédiction de l'indice de cétane de gazoles, comprenant les étapes consistant à :
a) mesurer le spectre I.R. de plusieurs ensembles de gazoles;
b) sélectionner dans une région spectrale une plage de constantes de longueur d'onde; et convertir un certain nombre des longueurs d'onde en question en données d'absorption, et utiliser lesdites données d'absorption comme entrées pour un réseau neural;
caractérisé par les étapes consistant à :
c) analyser des données spectrales en utilisant un réseau neural, le nombre des couches du réseau neural étant de 2 à 5, et dans le cas de trois couches ou davantage, le nombre des noeuds de la couche d'entrée est de 3 à 10, le nombre des noeuds de la ou des couches cachées est de 1 à 10 et le nombre des noeuds de la couche de sortie est de 1 à 3;
d) déterminer l'indice de cétane du gazole par une mesure classique;
e) sélectionner un ensemble de données d'apprentissage comprenant les données de mesure I.R. et les données d'indices de cétane mesurées de manière classique, et corréler les valeurs d'absorbance obtenues avec l'indice de cétane pour créer un ensemble de données prédictives; et ensuite
f) appliquer ces données sur des spectres infrarouges pris dans les mêmes conditions sur des gazoles dont l'indice de cétane est inconnu, pour ainsi fournir l'indice de cétane du gazole inconnu.

2. Procédé selon la revendication 1, dans lequel la plage spectrale est celle des longueurs d'onde de 0,78 à 30 µm.

3. Procédé selon la revendication 1 ou 2, dans lequel l'ensemble de gazoles en compte au moins 100.

4. Procédé selon l'une quelconque des revendications 1 à 3, dans lequel l'ensemble de gazoles contient des composants provenant de différents procédés de conversion secondaire.

5. Procédé selon l'une quelconque des revendications 1 à 4, dans lequel la région spectrale utilisée est de 9000 à 4500 constantes de longueur d'onde pour des gazoles prêts à l'usage et complètement mélangés, et la concentration en agents d'amélioration de l'allumage cétane est mesurée dans la région spectrale de 1600 à 1700 constantes de longueur d'onde.

6. Procédé selon l'une quelconque des revendications 1 à 4, dans lequel la région spectrale est de 9000 à 4500 constantes de longueur d'onde pour des courants de procédé de conversion présentant une plage d'indices de cétane.

7. Procédé selon l'une quelconque des revendications 1 à 6, dans lequel on utilise un réseau neural, comprenant les étapes consistant à :
effectuer l'apprentissage du réseau neural sur la totalité de l'ensemble de données en lui présentant de manière répétée des entrées et des sorties connues, c'est-à-dire les données dans l'infrarouge proche pour le gazole et son indice de cétane, pour qu'il apprenne la relation entre les deux, et surveiller les performances de cette prédiction par rapport aux données d'indices de cétane effectifs, mesurées par des procédés standard sur les données d'apprentissage, pour ainsi corréler les valeurs d'absorbance avec ledit indice de cétane;
créer un ensemble de valeurs des pondérations et déviations d'interconnexions du réseau, ajustées après ladite période d'apprentissage; et
appliquer ces valeurs ajustées en utilisant l'algorithme du réseau sur les spectres dans l'infrarouge proche, pris dans les mêmes conditions, pour des gazoles dont l'indice de cétane est inconnu.

8. Procédé selon l'une quelconque des revendications 1 à 7, dans lequel le réseau comprend 4 noeuds d'entrée, 2 noeuds cachés et un noeud de sortie (réseau (4, 2, 1)).

9. Procédé selon l'une quelconque des revendications 1 à 7, dans lequel le réseau est un réseau (3, 5, 1).

10. Procédé selon l'une quelconque des revendications 1 à 7, dans lequel le réseau est un réseau (6, 6, 3).

11. Procédé selon l'une quelconque des revendications 1 à 7, dans lequel le réseau est un réseau (6, 6, 6, 3).
